# FASCICULE DE BREVET EUROPEEN

(11) **EP 0 869 940 B1**
(45) Date de publication et mention de la délivrance du brevet: **05.04.2000**
(21) Numéro de dépôt: 96941102.4
(22) Date de dépôt: 03.12.1996
(51) Int. Cl.: C07C 319/24, C07C 321/14

(54) **PROCEDE DE PREPARATION DE DISULFURES ET DE POLYSULFURES ORGANIQUES EN PRESENCE DE RESINES POLYSTYRENE-DIVINYLBENZENE (PS-DVB) POSSEDANT DES GROUPES GUANIDINES OU AMIDINES**
VERFAHREN ZUR HERSTELLUNG ORGANISCHER DISULFIDE UND POLYSULFIDE IN GEGENWART VON POLYSTYROL-DIVINYLBENZOL (PS-DVB) RESINEN, DIE GUANIDIN- ODER AMIDIN GRUPPEN HABEN
METHOD FOR PREPARING ORGANIC DISULPHIDES AND POLYSULPHIDES IN THE PRESENCE OF POLYSTYRENE-DIVINYLBENZENE (PS-DVB) RESINS HAVING GUANIDINE OR AMIDINE GROUPS

(30) Priorité: 11.12.1995 FR 9514582
(43) Date de publication de la demande: 14.10.1998
(73) Titulaire: ELF AQUITAINE PRODUCTION, 92400 Courbevoie (FR)
(72) Inventeur: ARRETZ, Emmanuel, F-64000 Pau (FR); LOPEZ, Frédéric, F-64320 Idron (FR)
(74) Mandataire: Leboulenger, Jean
(86) Numéro de dépôt international: FR9601920
(87) Numéro de publication internationale: WO9721673

(56) Documents cités:
- EP-A- 0 337 837
- FR-A- 1 381 265
- US-A- 4 623 711
- US-A- 5 028 259
- J.M.S. - PURE APPL. CHEM., vol. A29, no. 3, 1992, pages 249-261, XP000196468 K. IIJIMA ET AL: cité dans la demande
- CHEMICAL ABSTRACTS, vol. 104, no. 12, 24 Mars 1986 Columbus, Ohio, US; abstract no. 90051k, XP002015445 & JP 60 177 006 A (MITSUBISHI CHEMICAL INDUSTRIES CO LTD)
- MAKROMOL. CHEM., vol. 185, no. 10, 1984, pages 2117-2124, XP000196458 M. TOMOI ET AL: cité dans la demande

## Description

La présente invention concerne la production de disulfures et de polysulfures organiques R-Sₙ-R (avec n ≥ 2) par réaction de mercaptans avec le soufre en présence de résines basiques agissant comme catalyseurs selon la réaction :

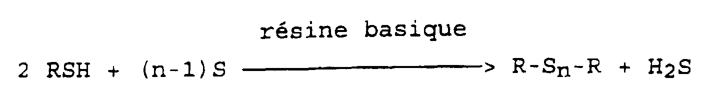

En présence de ces mêmes résines basiques, les disulfures et les polysulfures organiques de faible rang en soufre peuvent être transformés en polysulfures de rang supérieur en soufre par réaction avec le soufre. De même, en présence de ces mêmes résines basiques, les polysulfures organiques de rang élevé en soufre peuvent être transformés en polysulfures de rang inférieur en soufre par réaction avec des mercaptans.

Ainsi la demande de brevet EP-A-337 837 enseigne la préparation de disulfures et de polysulfures organiques en présence de résines organiques échangeuses d'anions comportant des groupes fonctionnels amine tertiaire ou ammonium quaternaire (actives sous forme OH⁻). De telles résines, généralement sous la forme de grains ou de billes insolubles dans les milieux réactionnels liquides et donc facilement séparables en fin de réaction, permettent d'obtenir des disulfures organiques et des polysulfures par réaction du soufre élémentaire avec des mercaptans et également d'obtenir des polysulfures organiques à rang élevé en soufre par réaction du soufre élémentaire sur des polysulfures organiques de rang inférieur en soufre.

S.V. Luis, M.I. Burguete et B. Altava, Reactive & Functional Polymers, 26, 1995, 75-83, indiquent que la chlorométhylation facile des résines polystyréniques et la haute réactivité des sites benzyliques résultants permet l'introduction d'un grand nombre de groupes fonctionnels et explique l'usage étendu de ces polymères.

Par contre, ces auteurs remarquent que la longueur réduite du bras espaceur méthylène réduit la mobilité des groupes fonctionnels introduits et, dans certains cas, rend leur accessibilité difficile aux réactifs, substrats et solvants. Cette situation peut conduire à une diminution de l'activité des groupes fonctionnels lorsqu'on les compare à leurs correspondants solubles. Dans certains cas, une amélioration nette de l'activité de ces groupes liés à la résine a été obtenue lorsque le site actif est séparé du squelette polymérique par un bras espaceur approprié.

S.V. Luis et al. préparent des résines polystyréniques ayant des bras espaceurs sous forme de chaîne aliphatique linéaire comportant 6 ou 9 groupes méthylène et portant en bout de chaîne un groupe hydroxyle -OH. Ce groupe hydroxyle est transformé en groupe partant tosylate, ce dernier étant remplacé par substitution par un groupe amine tertiaire.

Dans cette synthèse S.V. Luis et al. utilisent la fonctionnalisation de la résine polystyrénique par une réaction type Friedel Crafts à l'aide du chlorure d'acide dérivé d'un ester monoalkyl d'acide alcanedioïque.

Cette synthèse présente l'inconvénient majeur de la réduction à la fois d'un groupe tosylhydrazone et d'un groupe ester par de l'hydrure double (LiAlH₄) dans le tétrahydrofuranne (THF). Cette réduction rend cette voie de synthèse peu attractive sur le plan d'un développement industriel en des quantités importantes de résines comportant ces bras espaceurs en C₆ ou C₉.

D'autres auteurs se sont intéressés à l'obtention de bras espaceurs sous la forme d'une chaîne méthylène. Ainsi M. Tomoi, N. Kori et H. Kakiuchi, Reactive Polymers, 3, 1985, 341-349, introduisent sur des résines polystyréniques une longue chaîne aliphatique par alkylation à l'aide d'ω-bromoalkènes en présence d'acide trifluorométhanesulfonique.

Cependant cette synthèse est limitée à la préparation de polymères à bras espaceur, présentant un faible degré de réticulation (0-4 %).

Dans le document : Makromol. Chem 185, 1984, 2117-2124, M. Tomoi, Y. Kato et H. Kakiuchi ont effectué la synthèse de résines fonctionnalisées par un radical comprenant une amidine bicyclique : 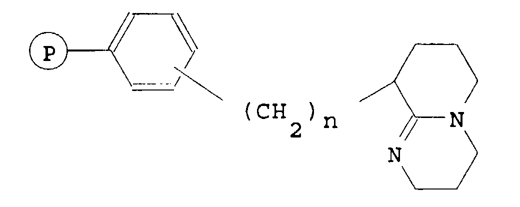 avec n entier valant 1, 4 ou 7.

Ces résines amidines (lorsque n vaut 4 ou 7) ont été préparées à partir des résines microporeuses ω-bromo alkylées, elles-mêmes obtenues à partir de ω-bromoalkylstyrène et de divinylbenzène.

D'une manière voisine dans le document :
J.M.S. Pure Appl. Chem., A29(3), pp 249-261 (1992), K. IIJima, W. Fukuda, et M. Tomoi ont effectué la synthèse d'une résine microporeuse fonctionnalisée par un radical comprenant une guanidine bicyclique :

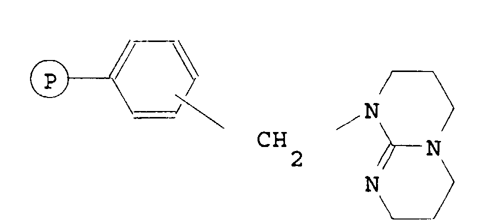

Ces auteurs ont étudié l'activité de la résine amidine bicyclique (n = 1) ci-dessus et de la résine guanidine ci-dessus dans l'estérification de l'acide benzoïque par le 1-bromobutane dans le toluène ou l'acétonitrile.

Ces deux résines se sont montrées efficaces dans l'alkylation de composés à méthylène actif par le 1-bromobutène.

De leur côté, G.D.Darling et M.J. Fréchet, J. Org. Chem., 51, 1986, 2270-2276, en partant d'une résine polystyrénique chlorométhylée ont obtenu un bras espaceur -(CH₂)₂- séparant la résine d'un hydroxyle -OH en bout de chaîne. Cet hydroxyle est transformé en tosylate, puis par la réaction de Gabriel, à l'aide de phtalimide de potassium et enfin par de d'hydrazine, en amine primaire. Cette synthèse a cependant l'inconvénient d'utiliser du n-butyllithium ou de l'hydrure double de lithium et d'aluminium.

L'objet de la présente invention est de proposer un procédé de préparation de disulfures et polysulfures organiques, selon les réactions exposées ci-dessus, en présence de résines PS-DVB fonctionnalisées et spécialement sélectionnées ou synthétisées afin d'obtenir de meilleurs résultats que dans l'art antérieur. Ces meilleurs résultats peuvent être, par exemple, un meilleur taux de conversion des réactifs et/ou une cinétique de réaction plus rapide.

Ce but est atteint par la mise en oeuvre de résines fonctionnalisées par des groupes guanidines ou amidines.

Plus précisément, la présente invention propose un procédé de préparation de disulfures et polysulfures organiques par réaction du soufre sur un mercaptan ou sur un polysulfure de rang inférieur en soufre pour le transformer en polysulfure de rang supérieur, ou encore par réaction d'un mercaptan sur un polysulfure organique de rang élevé en soufre pour le transformer en polysulfure de rang inférieur en soufre, en présence d'un catalyseur sous forme de résine à fonction basique, caractérisé en ce que la résine est à base de polystyrène-divinylbenzène (PS-DVB), fonctionnalisée par des groupes basiques et ayant la formule générale (I) : 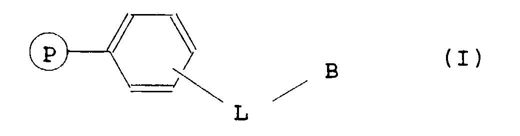 B représentant un groupe choisi parmi :
1° - un radical guanidine de formule générale (C) : 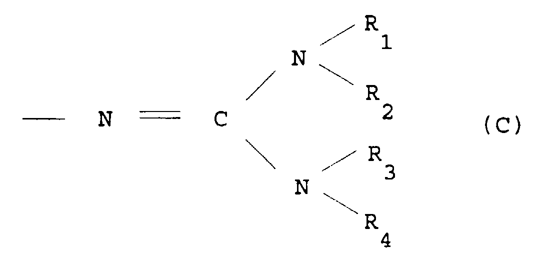 substitué par L sur l'azote imine, dans laquelle R₁, R₂, R₃ et R₄ sont indépendamment l'un de l'autre choisis parmi l'hydrogène, les groupes méthyle, éthyle, propyle, butyle, cyclohexyle, et phényle, avec la condition que L représente alors un radical organique linéaire ayant une longueur égale ou supérieure à celle du radical méthylène -CH₂-,
2° - un radical guanidine cyclique de formule (D) : 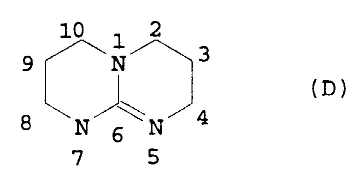 substitué par L en position 7, avec la condition que L représente alors un radical -(CH₂)ₙ-, n entier valant 1, 3 à 9,
3° - une amidine cyclique de formule (E) : 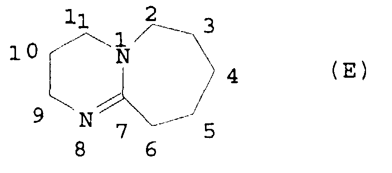 substituée par L en position 6, avec la condition que L représente alors un radical -(CH₂)ₙ-, n entier valant 1, 3 à 9,
4° - une amidine cyclique de formule (F) : 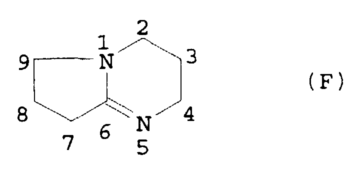 substituée par L en position 7, avec la condition que L représente alors un radical -(CH₂)ₙ-, n entier valant 1, 3 à 9 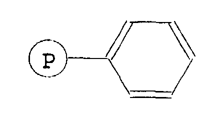 étant le support résine PS-DVB.

Les résines qui servent de matériaux de base pour la préparation des résines de formule générale (I) peuvent être des copolymères de PS-DVB ou des copolymères PS-DVB chlorométhylés.

Avec un faible taux de divinylbenzène (0,5 à 7% en poids) comme agent de réticulation, on obtient des copolymères du type gel, tandis qu'avec des teneurs plus élevées en DVB, on peut obtenir des résines macroréticulées et de structure macroporeuse.

Le taux de DVB peut être de 0,5 % à 60 % en poids par rapport au poids total du copolymère PS-DVB.

Les matériaux de base et, par voie de conséquence, les résines de formule générale (I) peuvent être de type gel, mais de préférence, les résines de formule générale (I) sont macroréticulées et de structure macroporeuse, car ces caractéristiques entraînent en général une meilleure activité catalytique dans le procédé.

Ces résines PS-DVB peuvent être chlorométhylées par l'éther chlorométhylique, selon des techniques connues et décrites dans la littérature, à des teneurs variables en chlore (Cl), généralement de 1 à 20 % en poids de chlore par rapport au poids de résine chlorométhylée.

De préférence, le radical L représente un méthylène. En effet, ces résines sont d'une synthèse aisée.

Avantageusement, le radical L a la formule générale(II) suivante :

-CH₂-(X-CH₂-CH₂)ₘ- (II),

dans laquelle X représente l'oxygène ou le soufre et m vaut 1 ou 2.

De préférence, dans la formule (II), X est l'oxygène et m vaut 1, ou encore X est le soufre et m vaut 1.

Avantageusement, les disulfures, polysulfures organiques et mercaptans ont des radicaux hydrocarbonés R choisis parmi un groupe alkyle, cycloalkyle, aryle aralkyle et alkylaryle.

La présente invention s'applique notamment à la production des disulfures et polysulfures de dialkyle contenant au total de 2 à 40 atomes de carbone, par exemple le disulfure et les polysulfures de diméthyle, diéthyle, dipropyle, dibutyle, dipentyle, dihexyle, diheptyle, dioctyle, didécyle et didodécyle. Elle s'applique également à la préparation de disulfures et polysulfures cycloalkyliques, par exemple, disulfure ou polysulfures de dicyclohexyle ou à la préparation par exemple de disulfure ou polysulfures de diphényle.

Avantageusement le radical hydrocarboné R porte un ou plusieurs groupes fonctionnels. Ces groupes sont, par exemple, les atomes d'halogène, -OH, -OR', -SR', NR'R'', CN, -CHO, -COR', -COOR', R' et R'' désignant des radicaux aliphatiques de C₁ à C₁₂, cycloaliphatiques, aromatiques ou alkylaromatiques.

L'activité catalytique des résines mises en oeuvre dans la présente invention, se manifeste à des teneurs très faibles de résines dans les mélanges.

Avantageusement, la résine est présente en une quantité allant de 0,01 à 20 parties en poids pour 100 parties en poids de mélange réactionnel, résine comprise.

Le procédé selon l'invention met en oeuvre une réaction qui peut être effectuée à une température de -10°C à 150°C. De préférence, la température est de +10°C à 120°C.

Les réactions peuvent être conduites à pression atmosphérique ou à des pressions supérieures pouvant atteindre 50 bars. En général, cette pression est de 28 bars absolu. Dans le cas de réactifs peu volatils et à faible tension de vapeur, la réaction peut être réalisée à des pressions inférieures à la pression atmosphérique, avec éventuellement la présence d'un gaz inerte, tel que l'azote.

Le rapport molaire mercaptan/soufre dépend de la nature du mercaptan utilisé et du produit à préparer (disulfure ou polysulfure). Avantageusement ce rapport est de 0,3 à 10 et de préférence de 0,4 à 6.

Dans le cas où l'on part d'un polysulfure organique de rang élevé en soufre que l'on veut transformer en polysulfure organique de faible rang en soufre, par exemple en trisulfure R-S₃-R ou disulfure R-S₂-R par action du mercaptan correspondant, avantageusement, on utilise un rapport molaire mercaptan/polysulfure allant de 2 à 10.

La production de disulfures ou de polysulfures organiques en présence des résines PS-DVB à fonction guanidine ou amidine peut être mise en oeuvre dans un réacteur agité ou tubulaire, suivant un procédé discontinu, soit par chargement des réactifs avant leur mise en réaction, soit par addition progressive de l'un des réactifs, soit, par addition simultanée des réactifs dans le réacteur, ou encore suivant un procédé continu avec addition contrôlée des réactifs.

Dans le cas où le soufre est l'un des réactifs (l'autre étant un mercaptan ou un polysulfure de faible rang en soufre), il peut être introduit sous la forme liquide ou solide.

Les résines de formule générale (I) peuvent être obtenues ou préparées de la manière suivante :

### 1° Le groupe B est un radical de formule générale (C).

On connaît du brevet US 5340380 un procédé consistant à substituer le chlore d'une résine chlorométhylée de polystyrène-divinylbenzène par une guanidine substituée ou non et permettant d'obtenir des résines de formule générale (I.C) : 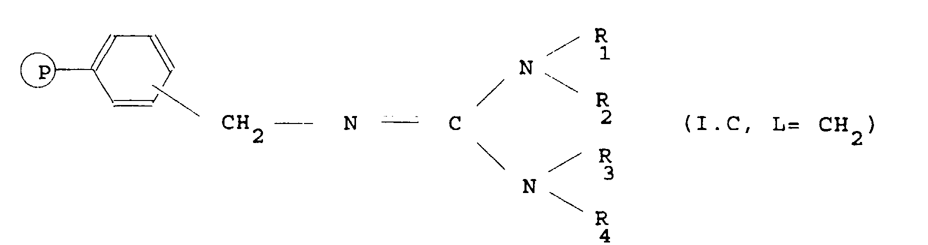 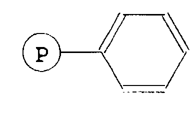 représentant le support solide, résine polystyrène-divinylbenzène de départ
R₁, R₂, R₃ et R₄ pouvant être chacun un hydrogène, un groupe alkyle ou un groupe aromatique.

Ainsi le brevet US 3346516 décrit cette technique de fonctionnalisation par réaction d'une résine polystyrène-divinylbenzène chlorométhylée avec la guanidine ou la tétraméthyl guanidine en présence d'un alcool inférieur et un solvant de gonflement du copolymère comme le tétrahydrofuranne, le dioxane ou le diglyme.

Dans le brevet US 5028259, la tétraméthylguanidine est mise en contact avec une résine chlorométhylée de polystyrène-divinylbenzène dans un mélange de toluène et de tétrahydrofuranne.

Dans le brevet US 5340380 des guanidines sont mises en réaction avec ce même type de résines chlorométhylées en présence de soude dans un solvant constitué d'éthanol ou d'eau.

Cependant cette technique de fonctionnalisation de résine PS-DVB chlorométhylée par une guanidine est très limitée dans la pratique pour l'obtention de résines de formules (I.C) dont les radicaux guanidines portent des substituants R₁ à R₄ différents de quatre méthyles ou de quatre hydrogènes, dans la mesure ou seules la guanidine et la 1,1,3,3,-tétraméthylguanidine sont actuellement commerciales.

De telles résines (I.C) dans lesquelles les groupes R₁ à R₄ sont tous différents de l'hydrogène peuvent être obtenues en utilisant des urées tétrasubstituées, souvent commercialisées, dans les conditions de préparation suivantes:
a) On commence par préparer une résine PS-DVB fonctionnalisée par des groupes amine primaire et ayant la formule générale (A) 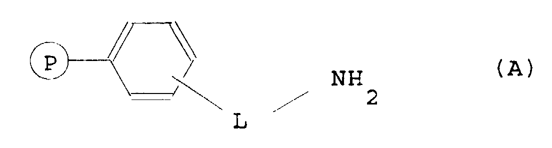 Celles-ci peuvent être obtenues par différentes techniques :
1/ On peut par exemple partir d'une résine de formule générale (J) : 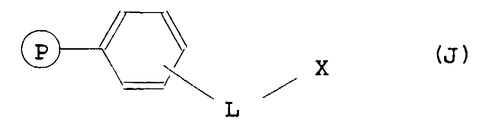 X étant un groupe partant notamment halogène ou tosylate obtenable à partir d'un groupe hydroxyle -OH, et L ayant la même signification que ci-dessus, notamment un radical -(CH₂)ₚ-, avec p entier valant de 1 à 9, y compris 2.

De préférence, lorsque L représente un seul méthylène, X est un atome de chlore. Dans ce cas, une méthode, décrite par D.H. Rich et S.K. Gurwara, J. Am. Chem. Soc., 1975, 97 - 1575-1579, consiste à faire réagir une résine PS-DVB chlorométhylée avec un excès d'ammoniac. Une autre voie est basée sur l'obtention de résine PS-DVB phtalimidométhylée qui est transformée par hydrazinolyse en résine à fonction amine primaire. Les deux méthodes d'accès à de telles résines phtalimido-méthylées sont décrites dans la publication de A.R. Mitchell, S.B.H. Kent, B.W. Erickson et R.B. Merrifield, Tetrahedron Letters N° 42, 1976, 3795-3798. L'une consiste à partir d'une résine PS-DVB qui par réaction avec le N-(chlorométhyl) phtalimide est directement convertie en résine phtalimidométhylée. L'autre méthode part d'une résine PS-DVB chlorométhylée qui est traitée par le phtalimide de potassium pour donner la résine correspondante phtalimidométhylée.

Quelques résines PS-DVB à fonction amine primaire de formule (A) dans laquelle L représente un méthylène sont commerciales.

Ainsi la Société PUROLITE propose deux résines macroporeuses l' A-107 et l' A-109, tandis que la Société FLUKA a, dans son catalogue 1995-1996, deux résines gels : la résine 08564 PS réticulée avec 2 % de DVB et contenant 1,1 mmole de groupe -NH₂ par gramme de résine, et la résine 08566 PS réticulée avec 1 % de DVB et contenant 0,6 mmole de -NH₂ par gramme de résine.

La méthode au phtalimide de potassium est également applicable aux résines de formule (J) dans le cas où L est un radical organique linéaire d'une longueur supérieure à celle du radical méthylène, notamment -(CH₂)ₙ-, avec n valant un nombre supérieur à 1.
2/ On peut également partir d'une résine PS-DVB de formule (J) dans laquelle L représente un méthylène et X a la signification ci-dessus et de préférence représente un atome de chlore. La Demanderesse a trouvé que cette résine chlorométhylée peut être mise en réaction avec une alkanolamine ou une mercaptoalkylamine, sous forme d'alcoolate alcalin, dans les conditions de la réaction de Williamson.

Si on met en oeuvre l'éthanolamine, on obtient des résines PS-DVB à fonction amine primaire avec des groupes fonctionnels -CH₂-O-CH₂-CH₂-NH₂ fixés aux supports résines PS-DVB.

De manière analogue, en partant du 2-amino éthanethiol hydrochlorure, on obtient les groupes fonctionnels -CH₂-S-CH₂-CH₂-NH₂.

Si on utilise le 2-(2-aminoéthoxy)éthanol, on obtient des résines PS-DVB à fonction amine primaire avec des groupes fonctionnels -CH₂(-O-CH₂-CH₂)₂-NH₂.

Enfin, en utilisant le 2-[(2-aminoéthyl)thio]-éthanethiol, on obtient des groupes fonctionnels -CH₂-(S-CH₂-CH₂)₂-NH₂.

Cette mercaptoalkylamine de départ peut être préparée selon Iwakura et al., J. Polym. Sci. Part A, 2, 1964, 881-883 ou selon I Voronkov, M.G. et al., Chem. Heterocycl. Compd. (Engl. Transl.)15, 1979, 1183-1185.

Les conditions générales de la réaction de Williamson sont les suivantes :

L'alcanolamine ou la mercaptoalkylamine diluée dans du tétrahydrofuranne (THF) anhydre, est mise à réagir avec de l'hydrure de sodium en suspension dans du THF anhydre. Après formation de l'alcanolate de sodium ou le mercaptide de sodium, la résine chlorométhylée est introduite dans le milieu réactionnel liquide.
b) Après obtention de la résine possédant des groupes amine primaire de formule générale (A), on fait réagir ces groupes avec du chlorure de chloroformamidinium (sel de Vilsmeier) de formule générale (H) : 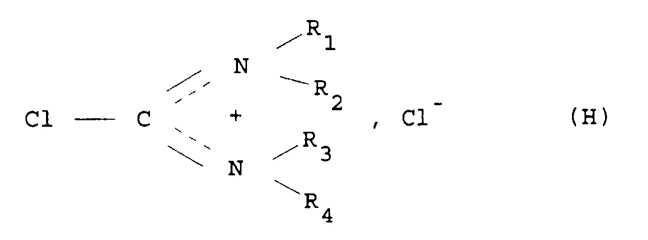 dans laquelle R₁, R₂, R₃ et R₄ sont indépendamment l'un de l'autre choisis parmi les groupes méthyle, éthyle, propyle, butyle, cyclohexyle, phényle,
pour obtenir une résine PS-DVB fonctionnalisée par un groupe guanidine et de formule générale (I.C) : 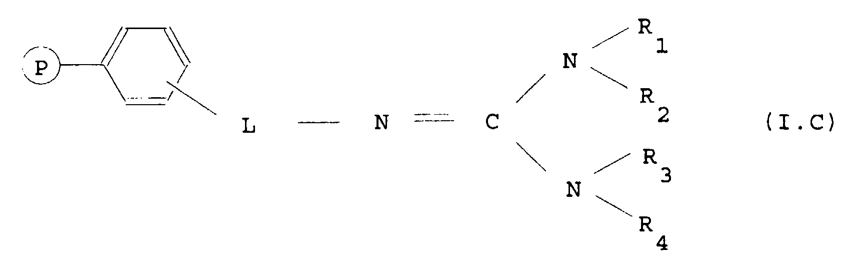 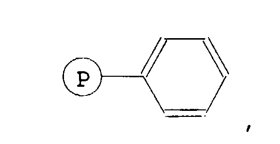 L, R₁ à R₄ ayant les mêmes significations que ci-dessus.

Les chlorures de chloroformamidinium (H) sont généralement obtenus à partir d'urées tétrasubstituées par réaction avec des composés électrophiles tels que phosgène, chlorure de thionyle, chlorure d'oxalyle, oxychlorure de phosphore, suivant des méthodes décrites dans la littérature, notamment:
- COCl₂: H. Eilingsfeld, M. Seefelder, Angew.Chem., 72, 1960, 836.
- SOCl₂: H. Ulrich, A.A.R. Sayigh, Angew. Chem. Intern. Ed. Engl., 5, 1966, 704.
- (COCl)₂: T. Fujisawa et al., Chem. Lett.,1982, 1891.
- POCl₃: H. Bredereck, K. Bredereck, Chem. Ber.,94, 1961, 2278.

Généralement, on part de quantités stoechiométriques d'urées tétrasubstituées et de composés chlorés électrophiles et on opère en présence d'un solvant tel que le tétrachlorure de carbone dans le cas du chlorure d'oxalyle, soit sans solvant avec le phosgène ou le chlorure de thionyle, à une température généralement de 0°C à 40°C, et en atmosphère anhydre pour éviter toute hydrolyse.

Avantageusement, on choisit les urées tétrasubstituées parmi la tétraméthylurée, la tétraéthylurée, la tétra n-propylurée et la tétra n-butylurée.

Les chlorures de chloroformamidinium (H) sont mis généralement dans un solvant tel que le toluène ou l'acétonitrile. Leurs réactions avec les résines à fonction amine primaire (A) sont effectuées en présence d'une base, de préférence en présence d'un excès de base.

Si la base est la triéthylamine (TEA), on opère généralement avec un excès molaire de TEA de 10 à 50% par rapport aux chlorures de chloroformamidinium (H). Ces derniers sont généralement en excès molaire de 10 à 100% par rapport au nombre de moles de fonction amine primaire, pour transformer la totalité de ces dernières en fonction guanidine.

### 2° Le groupe B est un radical de formule générale (D)

(a) On commence par préparer une résine de formule générale (J) comme au point 1° a) ci-dessus, L représentant un radical -(CH₂)ₙ-, n entier valant 1, 3 à 9 et X étant un chlore ou un brome.
(b) La résine halogénée ci-dessus est mise en réaction avec du 1,5,7-triaza-bicyclo [4.4.0] déc-5-ène (TBD), d'une manière analogue au procédé de M. Tomoi et al, J.M.S. Pure Appl. Chem. A29(3), 1992, 249-261, en particulier page 251 (" Preparation of Polystyrene-Supported TBD).
   On obtient ainsi une résine PS-DVB fonctionnalisée par un groupe guanidine bicyclique de formule générale (I.D.) : 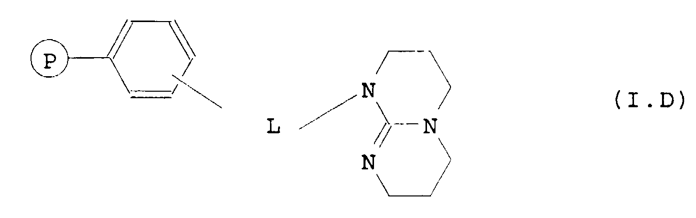 L représentant un radical -(CH₂)ₙ- avec n entier valant 1, 3 à 9.

### 3° Le groupe B est un radical de formule générale (E)

a) on commence par préparer la résine de formule générale (J) comme au point 2° a) ci-dessus.
b) La résine halogénée obtenue ci-dessus est mise en réaction avec du 1,8-diazabicyclo [5.4.0]undec-7-ène (DBU) d'une manière analogue au procédé de M. Tomoi et al., Makromol. Chem, 185, 1984, 2117-2124, en particulier page 2118, " Preparation of polystyrene-supported DBU).
   On obtient ainsi une résine PS-DVB fonctionnalisée par un groupe amidine bicyclique de formule générale (I.E.) : 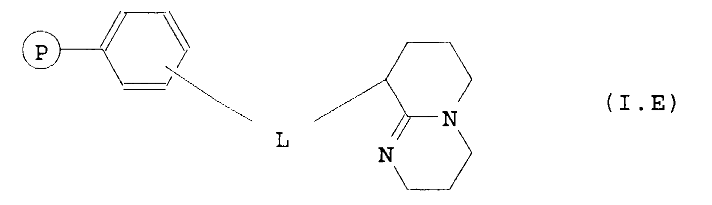 L représentant un radical -(CH₂)ₙ- avec n entier valant 1, 3 à 9.

### 3° Le groupe B est un radical de formule générale (F)

On procède comme en 2° a) et 2° b) sauf que l'on remplace le DBU par du 1,5-diazabicyclo[4.3.0]non-5-ène (DBN).
On obtient ainsi une résine PS-DVB fonctionnalisée par un groupe amidine bicyclique de formule générale (I.F.): 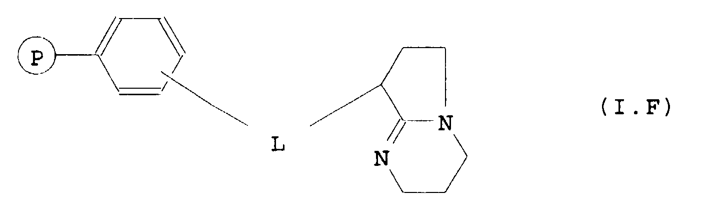 L représentant un radical -(CH₂)ₙ- avec n entier valant 1, 3 à 9.

L'efficacité catalytique des résines utilisées dans la présente invention se trouve améliorée lorsqu'on les utilise sèches.

L'activité catalytique des résines de type gels, utilisées dans la présente invention, est fortement améliorée par la présence de méthanol dans les mélanges réactionnels tels que rapportés dans le préambule de la revendication 1 de procédé.

Cet effet promoteur du méthanol (observé aussi avec l'éthanol, mais à un niveau moins important) se manifeste à partir de faibles teneurs dans les mélanges réactionnels, pour des quantités de méthanol allant de 0,01 g à 2 g pour 100 g de mélange réactionnel comprenant résine, soufre, mercaptan et méthanol ou résine soufre, polysulfure et méthanol, ou encore résine, mercaptan, polysulfure et méthanol. Ainsi, avantageusement on ajoute du méthanol au milieu réactionnel.

En plus de la description qui précède, la présente invention sera mieux comprise à l'aide de la partie expérimentale qui suit à titre illustratif.

### Partie expérimentale

Les séchages des résines sont pratiqués sous un vide d'environ 4x10³ Pascal(Pa).

### 1° Obtention de résines PS-DVB de formule (I.C) à fonction 1,1,3,3-tétraméthyl-guanidine (TMG) (L = -CH₂-, R₁=R₂=R₃=R₄=CH₃-).

La technique utilisée consiste à incorporer directement à une résine PS-DVB chlorométhylée la TMG, selon la méthode décrite dans les brevets US 3346516 et 5028259.

Deux types de résines PS-DVB chlorométhylées sont utilisées :
a) une résine de type gel : Bio-Beeds® SSX1, à base de PS réticulé à 1% de DVB et chlorométhylée avec un taux de chlore de 11,52% en poids par rapport au poids total. Cette résine contient donc 4,09 mmoles de Cl/g de résine.
b) Une résine de type macroporeux qui a les caractéristiques suivantes:
   Surface spécifique : 22,5 m²/g de résine
   Diamètre moyen des pores : 20 Å
   Volume des pores : 69%
   chlorométhylée avec un taux de chlore de 19,32% en poids par rapport au poids total.
   Cette résine contient donc 5,44 meq de Cl/g de résine.

### Mode opératoire :

On pèse une quantité déterminée de résine chlorométhylée sèche contenant 0,0544 mole de Cl (soit 13,3 g pour la résine 1° a) et 10 g pour la résine 1° b)) et on la met en contact sous atmosphère d'azote avec 12,5 g (0,1088 mole) de TMG dilués dans 130 ml de tétrahydrofuranne (THF) préalablement séché sur tamis moléculaire. Le milieu réactionnel ainsi obtenu est agité mécaniquement pendant 48 heures à une température de 60°C. Après refroidissement à 20°C, on filtre la résine et on la lave au THF, puis avec 200 ml d'une solution aqueuse de soude à 10% et enfin à l'eau jusqu'à neutralité. La résine est lavée à l'acétone puis séchée sous vide à 60°C à poids constant.

On a effectué l'analyse élémentaire des deux résines ainsi obtenues.

Pour la résine a) de type gel référencée par la suite N°1 (TMG), N = 7,85% en poids soit 1,87 mmole de TMG/g de résine.

Pour la résine b) de type macroporeux référencée par la suite N°2 (TMG), N = 8,74% en poids, soit 2,08 mmole de TMG/g de résine.

### 2° Obtention de résines PS-DVB de formule (I.C) à fonction TMG (L = -CH₂-O-CH₂-CH₂-, R₁=R₂=R₃=R₄=-CH₃). Les deux résines a) et b) ci-dessus ont été utilisées.

a) Préparation de l'aminoéthanolate de sodium (NaO-(CH₂)₂-NH₂).
   On additionne lentement sous atmosphère d'azote une solution de 2,5 ml d'éthanolamine dans 25 ml de THF anhydre (fraîchement distillé sur sodium) à une solution de 1,5 g d'hydrure de sodium dissous dans 40 ml de THF anhydre. On maintient le milieu réactionnel sous agitation à la température de 20°C pendant 1 heure puis on le porte au reflux pendant 2 heures. On refroidit ensuite la solution d'aminoéthanolate de sodium à 20°C en la maintenant sous atmosphère d'azote.
b) Obtention de résines de formule générale (A) à fonction amine primaire, dans laquelle L représente -CH₂-O-CH₂-CH₂-;
   On sélectionne une quantité déterminée de la résine ci-dessus 1° a) chlorométhylée sèche contenant 0,0272 mole de chlore, soit 6,65 g.
   On pratique la même sélection avec la résine 1° b) chlorométhylée sèche sur la base de 0,0272 mole de chlore, soit 5 g.
   On traite séparément chacune de ces résines de la manière suivante :
   On introduit la résine sélectionnée (6,65 ou 5 g) dans une solution d'aminoéthanolate préparée selon 2° a). On maintient le milieu sous agitation à 70°C pendant 48 heures. Après refroidissement vers 20°C on récupère la résine. On la lave à l'eau puis avec une solution aqueuse à 5% en poids de potasse, ensuite à l'eau jusqu'à neutralité. La résine humide est lavée au méthanol et enfin séchée sous vide à 60°C.
c) Préparation du chlorure de tétraméthylchloroformamidinium
   On verse goutte à goutte une solution de 2,5 ml (0,027 mole) de chlorure d'oxalyle dans 15 ml de tétrachlorure de carbone anhydre, à une solution de 3,4 ml (0,027 mole) de tétraméthylurée dans 10 ml de tétrachlorure de carbone anhydre maintenue agitée et sous atmosphère d'azote. Toujours sous agitation, on porte le milieu réactionnel au reflux jusqu'à la fin du dégagement gazeux de dioxyde de carbone et de monoxyde de carbone. Le chlorure de formamidinium obtenu sous la forme d'un solide blanc est solubilisé à 0°C dans 30 ml d'acétonitrile anhydre.
d) Réaction avec les résines ci-dessus à fonction amine primaire (L = -CH₂-O-CH₂-CH₂-)
   Pour chacune des deux résines ci-dessus, on met en suspension, sous agitation, à la température de 0°C, 5 g de résine dans un mélange de 5,2 ml (0,0374 mole) de triéthylamine et 20 ml d'acétonitrile anhydre. A cette suspension maintenue à 0°C on additionne lentement la solution obtenue ci-dessus de chlorure de formamidinium dans 30 ml d'acétonitrile anhydre. On agite pendant 72 heures à la température de 20°C puis on porte au reflux pendant 1 heure. Après refroidissement à 20°C, on filtre la résine, on la lave à l'eau puis avec une solution de soude à 10% en poids, de nouveau à l'eau jusqu'à neutralité. La résine humide est lavée au méthanol, puis à l'acétone et enfin séchée sous vide à 60°C.
   On pratique des analyses élémentaires sur les deux résines ainsi obtenues et on calcule le nombre de mole de fonctions guanidine greffées :
   - pour la résine de type gel issue de 1° a) :
      N = 9,03% soit 2,15 mmoles de tétraméthylguanidine (-TMG)/g de résine, référencée par la suite N°3 (-TMG).
   - pour la résine de type macroporeux issue de 1° b) :
      N = 6,98% soit 1,66 mmole de tétraméthylguanidine (-TMG)/g de résine, référencée par la suite N°4 (-TMG).

### 3° Obtention de résines PS-DVB de formule (I.C) à fonction tétra, n-butyl-1,1,3,3-guanidine (TBG) (L = -CH₂-, R₁ = R₂ = R₃ = R₄ = n-Butyl).

La technique de préparation utilisée consiste à partir d'une résine PS-DVB non fonctionnalisée. Ce copolymère est dans une première étape fonctionnalisé en résine aminométhylée (fonction -CH₂-NH₂) selon la méthode décrite dans Tetrahedron Letters N°42, 1976, 3795-3798. La résine obtenue est ensuite fonctionnalisée en résine à fonction tétra, n-butyl-1,1,3,3 guanidine par le chlorure de tétra, n-butylchloroformamidinium.

La résine PS-DVB utilisée est un copolymère synthétique poreux fabriqué par Rohm and Haas, l'Amberlite XAD-4. Les caractéristiques de cette résine fortement réticulée, données par Rohm and Haas sont :
Surface spécifique : 750 m²/g de résine
Diamètre moyen de pore : 50 Å
Volume des pores : 51%.
a) Fonctionnalisation de la résine Amberlite XAD-4 en résine phtalimidométhylée.
   On met en suspension 10 g de résine Amberlite XAD-4 préalablement séchée dans une solution de 0,5 ml (0,0043 mole) de tétrachlorure d'étain dans 30 ml de 1,2-dichloro-éthane. A cette suspension, on ajoute sous agitation et à 60°C, 6,7 g (0,0342 mole) de N-chlorométhyl phtalimide en solution dans 20 ml de 1,2-dichloroéthane. Le milieu réactionnel est maintenu au reflux (82-84°C) sous agitation pendant 5 heures. Après retour à la température ambiante (20°C), on filtre la résine, puis on la lave au dichloroéthane et enfin au méthanol. Après séchage à 60°C sous vide, on obtient 13,1 g de résine.
   Spectre IR : υ (CO-N-CO) et δ (CO-N-CO) à 1770 cm⁻¹ et 1710 cm⁻¹.
b) Hydrazinolyse pour l'obtention de la fonction amine primaire.
   A une suspension de 12 g de la résine obtenue en 3° a) dans 40 ml d'éthanol absolu, sous agitation, on ajoute 4,5 ml (0,092 mole) d'hydrate d'hydrazine et 0,9 g (0,022 mole) de soude en pastilles.
   On porte ensuite le milieu réactionnel au reflux pendant 48 heures. Après refroidissement à 20°C, on filtre la résine, on la lave à l'éthanol puis avec une solution aqueuse de potasse à 5% en poids. On lave ensuite la résine à l'eau jusqu'à neutralité, à l'éthanol, à l'acétone et enfin au méthanol. On la sèche ensuite sous vide à 60°C pour obtenir 11 g de résine. Cette résine ne montre plus en IR les bandes caractéristiques du phtalimide.
   On effectue une analyse élémentaire :
   N = 3,53% soit 2,52 mmole de -NH₂/g de résine.
c) Fonctionnalisation de cette résine aminométhylée en fonction tétra-n-butylguanidine.
   On prépare du chlorure de tétra, n-butyl chloroformamidinium en faisant barboter 5,5 g de phosgène pendant 2 heures dans 10,4 g (0,037 mole) de tétra, n-butylurée, sous agitation, à la température de 80°C, pendant 5 heures. On élimine ensuite l'excès de phosgène par évaporation sous vide. On obtient 12,45 g de chlorure de tétra, n-butylchloroformamidinium sous la forme d'un solide blanc.
   Ce chlorure est solubilisé dans 40 ml d'acétonitrile anhydre.
   A cette solution de chlorure maintenue agitée, on additionne sous atmosphère d'azote et à 0°C, 10 g de la résine aminométhylée et 8,7 ml (0,0625 mole) de triéthylamine. On maintient le mélange réactionnel sous agitation à 20°C pendant 96 heures, puis on le porte au reflux pendant 1 heure. Après refroidissement à 20°C, on filtre la résine, on la lave à l'acétonitrile puis avec une solution aqueuse de soude à 10% en poids. On la lave ensuite à l'eau jusqu'à neutralité, à l'acétone, au méthanol et à nouveau à l'acétone. On la sèche sous vide à 60°C pour obtenir 11,3 g de résine.
   On effectue une analyse élémentaire :
   N = 4,62% soit 1,1 mmole de guanidine (-TBG)/g de résine.
   Cette résine est référencée par la suite N°1 (-TBG).

### 4° Obtention de résines PS-DVB de formule (I.D.) à fonction 1,5,7-triazabicyclo[4.4.0] déc-5-ène (TBD) avec L = -CH₂-.

Le procédé utilisé est décrit dans J. Macromol. Sci. Pure. Appl. Chem A29(3) 1992, 249-263.

Il consiste à faire réagir le sel de lithium du 1,5,7-triazabicyclo[4.4.0]déc-5-ène avec une résine PS-DVB chlorométhylée.
a) Préparation du sel de lithium du TBD.
   On additionne 0,052 mole de n-butyllithium dans 25 ml d'hexane, à un mélange de 7,9 g (0,057 mole) de TBD et de 250 ml de THF anhydre, à une température de -78°C et sous atmosphère d'argon. On agite ensuite le milieu réactionnel à -78°C pendant 2 heures.
b) Obtention des résines (I.D.) à fonction TBD.
   On pèse une quantité déterminée de résine chlorométhylée correspondant à 0,0544 mole de chlore (soit 13,3 g de la résine type gel 1° a) ou 10 g de la résine de type macroporeux 1°b)). Cette quantité est ajoutée lentement sous atmosphère d'argon à la solution du sel de lithium du TBD maintenue à -78°C.
   Sous agitation, on porte ensuite progressivement le mélange réactionnel à 20°C et on le maintient ainsi pendant 70 heures. On ajoute ensuite 30 ml de méthanol et on filtre la résine sur verre fritté. On la lave successivement avec un mélange THF/méthanol 1/1 en volume, puis au méthanol, avec un mélange méthanol/eau 1/1 en volume, à l'acétone, au THF, au dichlorométhane. On sèche la résine sous vide à 60°C.
   On effectue des analyses élémentaires des deux résines ainsi obtenues.
   - Résine de type gel :
      N = 11,88% soit 2,83 mmoles de fonction TBD/g de résine
      Cette résine est référencée par la suite N°1 (-TBD).
   - Résine de type macroporeux :
      N = 11,29% soit 2,69 mmoles de fonction TBD/g de résine
      Cette résine est référencée par la suite N°2 (-TBD).

### Exemple 1 Production de di, n-butyldisulfure par réaction du n-butylmercaptan avec le soufre.

On effectue des essais de production du di, n-butyl disulfure dans des conditions expérimentales identiques à l'aide des résines à fonction guanidine (TMG) ou guanidine bicyclique (TBD).

On utilise également une résine commerciale Fluka à fonction TBD, L = -CH₂-:
N°90603 du catalogue Fluka 1995/96
PS réticulé à 2% de DVB
TBD : 2,8 mmoles de TBD/g de résine sèche.

On effectue également des essais comparatifs à l'aide d'une résine PS-DVB de type macroporeux à fonction amine tertiaire, L = -CH₂-, la résine Amberlyst A-21 fabriquée par la Société Rohm and Haas.
Caractéristique de la résine Amberlyst A-21 :
Surface spécifique : 39,8 m²/g de résine fonctionnalisée par -CH₂-N(CH₃)₂ : 4,4 mmoles de fonction amine tertiaire/g de résine sèche.

On effectue les essais dans un réacteur constitué d'un erlenmeyer en verre d'un volume de 50 ml à deux tubulures muni d'un réfrigérant à circulation d'eau et d'une gaine thermométrique pour la mesure de la température du milieu réactionnel. Ce réacteur est chauffé par un bain d'huile placé sur la plaque d'un agitateur magnétique chauffant. L'agitation est obtenue dans le réacteur par un aimant teflonné.

### Mode opératoire :

On introduit dans le réacteur 26,58 g (0,295 mole) de n-butylmercaptan, 4,5 g (0,147 mole) de soufre solide finement broyé et 0,1 g de résine sèche. Sous agitation on porte le milieu réactionnel à 60°C. Après disparition totale du soufre solide (généralement au bout de 15 min.) on prélève à des temps déterminés des échantillons du milieu réactionnel liquide. Ces échantillons sont analysés par chromatographie en phase gazeuse sur une colonne capillaire Hewlett-Packard Ultra-1 de 50 m de long pour en déterminer la teneur en di, n-butyldisulfure (S₂%) en poids formé en fonction du temps en min.

Dans le cas des essais effectués en présence de méthanol, la quantité de méthanol incorporé dans le mélange réactionnel est de 0,4 g (0,0125 mole). Les résultats figurent dans les Tableaux I et II suivants.

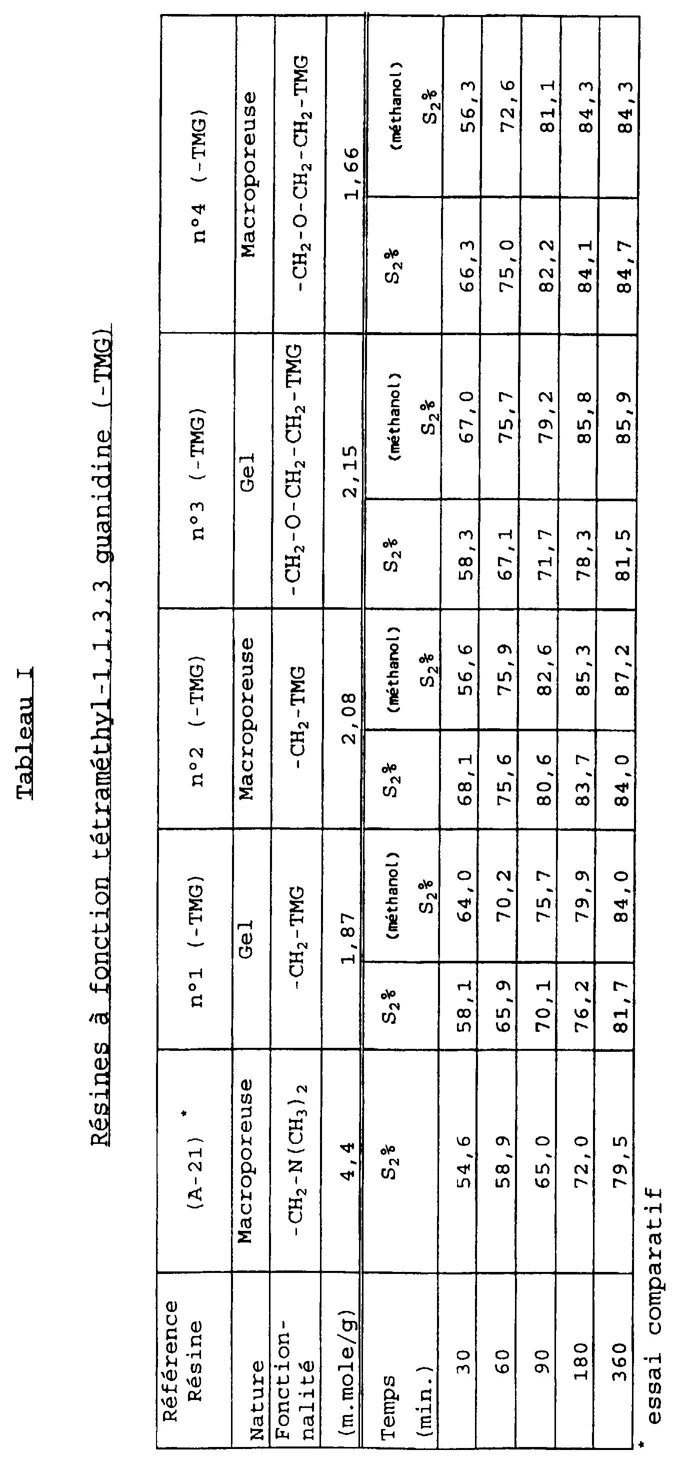

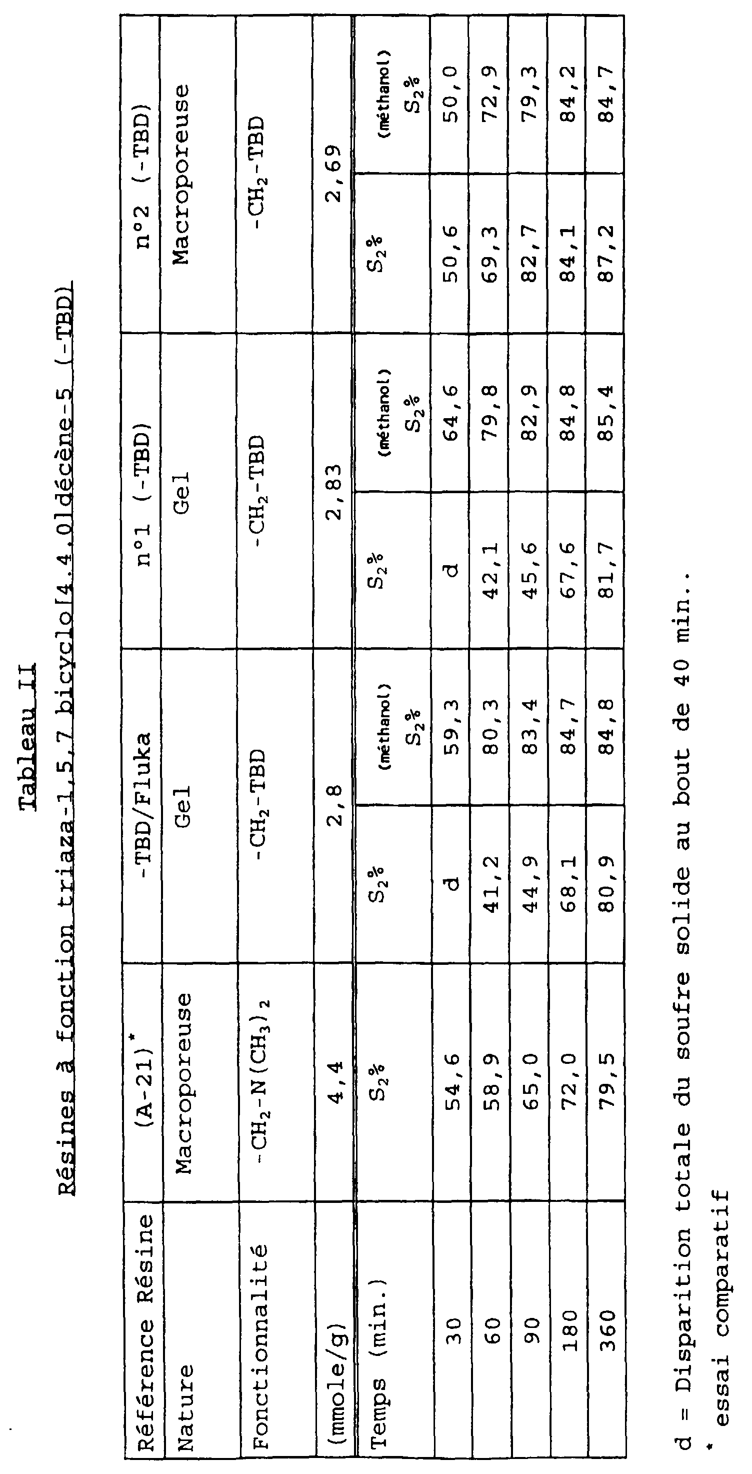

Ces tableaux montrent que le procédé selon la présente invention conduit à de meilleurs résultats que l'art antérieur.

### Exemple 2 Production de di, tert-butylpolysulfures par réaction du tert-butylmercaptan avec le soufre

On effectue des essais de production de polysulfures à partir de tert-butylmercaptan et de soufre en présence de résines et éventuellement de méthanol. Un essai comparatif est effectué avec la résine Amberlyst A-21.

### Mode opératoire :

Ces essais sont réalisés dans le même appareillage que dans l'Exemple 1, dans les conditions identiques suivantes :

On introduit 26,5 g (0,294 mole) de tert-butylmercaptan avec 18 g (0,56 mole) de soufre finement broyé dans le réacteur et sous agitation on porte le milieu réactionnel à 60°C.

On note la durée de la disparition totale du soufre. Après 90 min. de réaction, on prélève un premier échantillon du milieu réactionnel liquide, suivi d'autres prélèvements successifs au cours du temps. Les échantillons sont analysés par chromatographie en phase gazeuse sur une colonne capillaire Hewlett-Packard Ultra-1 de 50 m de long pour déterminer leur teneur en tert-butylmercaptan restant, représentative de la vitesse de ce mercaptan en polysulfures correspondants.

Le tableau III suivant montre les résultats de ces essais, qui donnent pour chaque résine testée le temps au bout duquel tout le soufre solide a disparu et la teneur en tert-butylmercaptan (TMB % en poids) restant dans le milieu réactionnel.

Dans le cas des essais effectués en présence de méthanol, la quantité de cet alcool incorporé au milieu réactionnel est de 0,4 g (0,0125 mole).

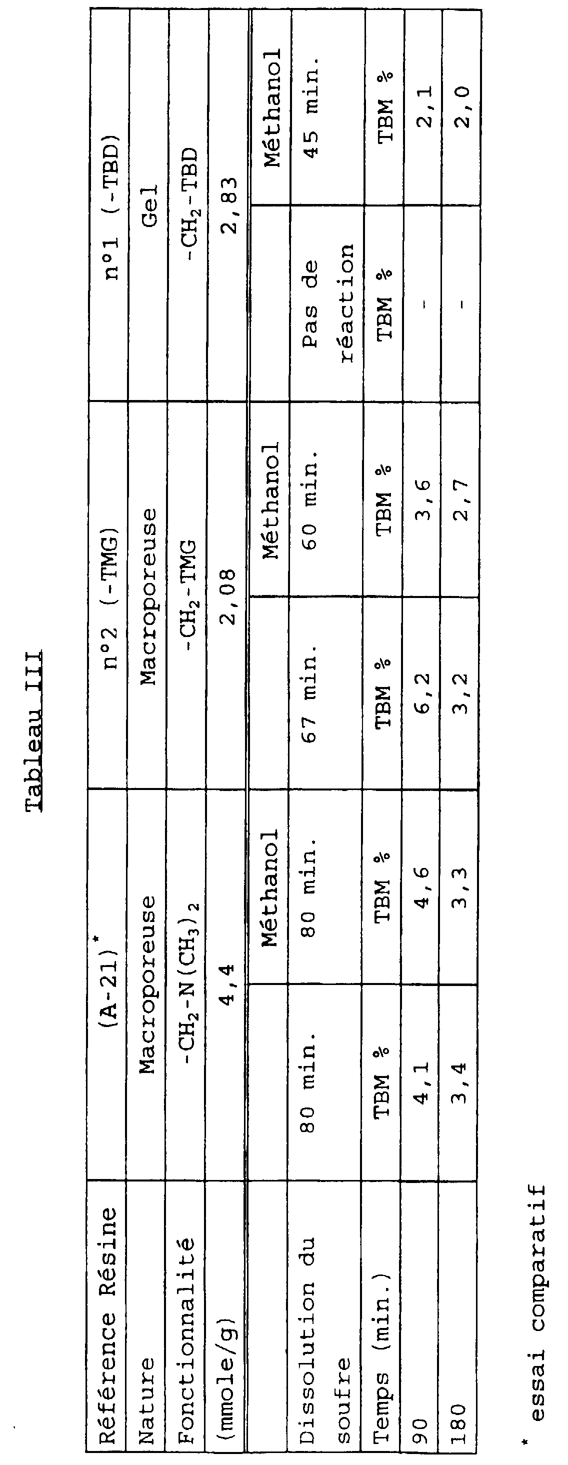

On remarque que les résultats du procédé selon l'invention sont meilleurs que celui de l'art antérieur.

### Exemple 3 Production de di, tert-butyltrisulfure par rétrogradation par le tert-butylmercaptan de di, tert-butylpolysulfures de rangs plus élevés en soufre

Le di, tert-butylpolysulfure utilisé a une masse molaire moyenne de 250 et une teneur en soufre de 54,4% et une teneur en di, tert-butyltrisulfure, déterminée par chromatographie en phase gazeuse, de 29,5% sur une colonne capillaire Hewlett-Packard Ultra-1 de 50 m de long, le complément est constitué de polysulfures de rangs plus élevés en soufre.

Les essais de rétrogradation des polysulfures (Sₓ avec x > 3) par le tert-butylmercaptan sont effectués en présence de différentes résines guanidine en présence éventuellement de méthanol.

Un essai comparatif est effectué avec la résine Amberlyst A-21.

Tous les essais sont réalisés dans un appareillage identique à celui précédemment décrit.

### Mode opératoire :

On introduit dans le réacteur 10 g (0,0365 mole) de di, tert-butylpolysulfure et 19,71 g (0,219 mole) de tert-butylmercaptan et 0,5 g de la résine choisie. Le milieu réactionnel est porté rapidement à la température de 60°C, sous agitation. A intervalles de temps déterminés, on prélève des échantillons qui sont analysés par chromatographie en phase gazeuse sur une colonne capillaire Hewlett-Packard Ultra-1 de 50 m de long.

Le suivi chromatographique permet de déterminer la teneur en di, tert-butyltrisulfure formé au cours du temps.

Dans le cas des essais effectués en présence de méthanol, la quantité de cet alcool incorporé dans le milieu réactionnel est de 0,4 g (0,0125 mole).

Les résultats des essais avec les différentes résines testées figurent dans les Tableaux IV, V et VI suivants, qui donnent pour chaque résine testée la proportion en poids de di, tert-butyltrisulfure (S₃%) formé en fonction du temps en min.

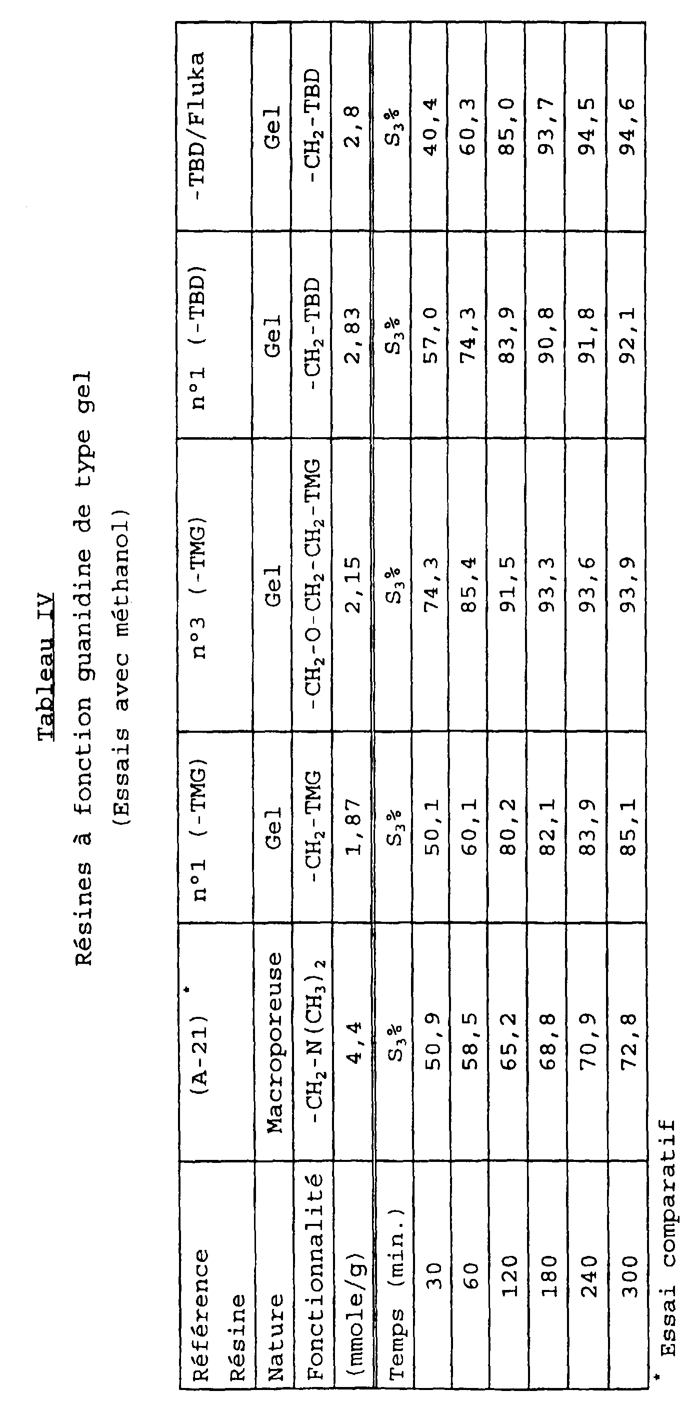

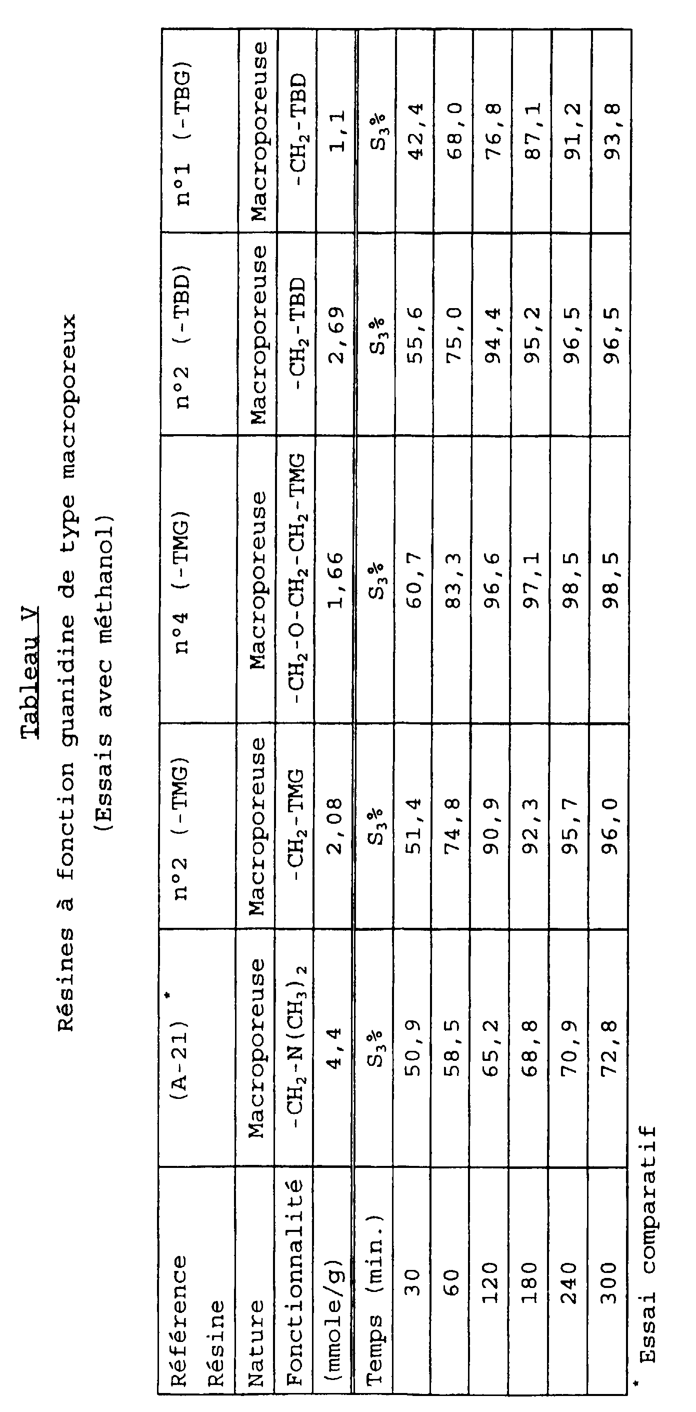

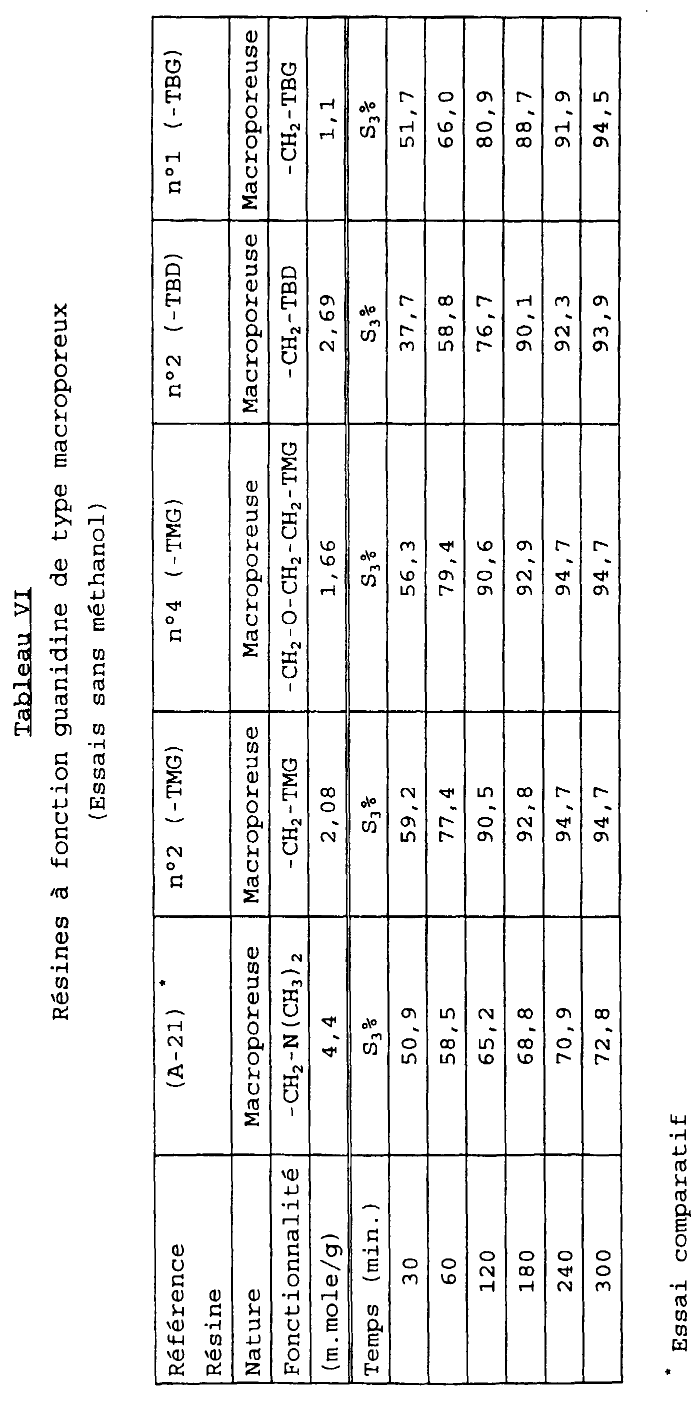

On remarque de ces résultats que le méthanol n'a aucun effet positif sur la réaction en présence de la résine Amberlyst A-21 (Essai comparatif).

Dans cette réaction de rétrogradation des di, tert-butylpolysulfures en di, tert-butyltrisulfure, les résines à fonction guanidine de type gel, ont des réactivités très faibles en l'absence de méthanol. Par contre de manière inattendue, la présence de méthanol, en faible proportion, a un effet promoteur très important. L'effet du méthanol est beaucoup plus atténué dans le cas des résines à fonction guanidine de type macroporeux.

## Revendications

1. Procédé de préparation de disulfures et polysulfures organiques par réaction du soufre sur un mercaptan ou sur un polysulfure de rang inférieur en soufre pour le transformer en polysulfure de rang supérieur, ou encore par réaction d'un mercaptan sur un polysulfure organique de rang élevé en soufre pour le transformer en polysulfure de rang inférieur en soufre, en présence d'un catalyseur sous forme de résine à fonction basique, caractérisé en ce que la résine est à base de polystyrène-divinylbenzène (PS-DVB) fonctionnalisée par des groupes basiques et ayant la formule générale (I) : 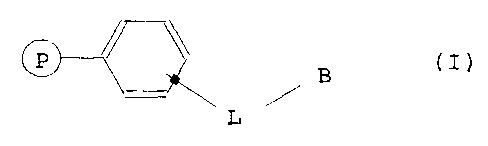 B représentant un groupe choisi parmi :
1° - un radical guanidine de formule générale (C) : 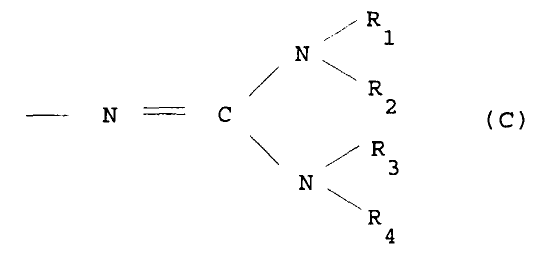 substitué par L sur l'azote imine, dans laquelle R₁, R₂, R₃ et R₄ sont indépendamment l'un de l'autre choisis parmi l'hydrogène, les groupes méthyle, éthyle, propyle, butyle, cyclohexyle, et phényle, avec la condition que L représente alors un radical organique linéaire ayant une longueur égale ou supérieure à celle du radical méthylène -CH₂-,
2° - un radical guanidine cyclique de formule (D) : 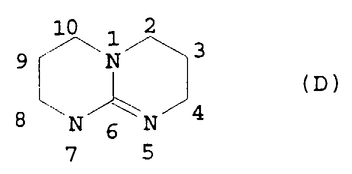 substitué par L en position 7, avec la condition que L représente alors un radical -(CH₂)ₙ-, n entier valant 1, 3 à 9,
3° - une amidine cyclique de formule (E) : 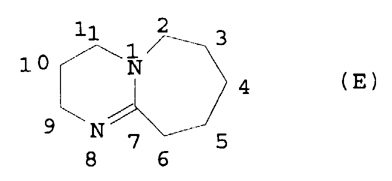 substituée par L en position 6, avec la condition que L représente alors un radical -(CH₂)ₙ-, n entier valant 1, 3 à 9,
4° - une amidine cyclique de formule (F) : 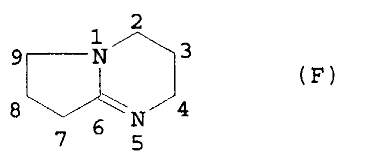 substituée par L en position 7, avec la condition que L représente alors un radical -(CH₂)ₙ-, n entier valant 1, 3 à 9 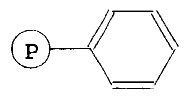 étant le support résine PS, DVB.

2. Procédé suivant la revendication 1, caractérisé en ce que la résine de formule générale (I) est de type gel.

3. Procédé suivant la revendication 1, caractérisé en ce que la résine de formule générale (I) est macroréticulée et de structure macroporeuse.

4. Procédé suivant l'une des revendications 1 à 3, caractérisé en ce que L représente un méthylène (-CH₂-).

5. Procédé suivant l'une des revendications 1 à 3, caractérisé en ce que lorsque L est rattaché au radical guanidine de formule (C) il représente un radical de formule (II)
-CH₂-(X-CH₂-CH₂)ₘ- (II)
X représentant l'oxygène (-O-) ou le soufre (-S-) et m valant 1 ou 2.

6. Procédé suivant la revendication 5, caractérisé en ce que X représente l'oxygène et que m vaut 1.

7. Procédé suivant la revendication 5, caractérisé en ce que X représente le soufre et que m vaut 1.

8. Procédé suivant l'une des revendications 1 à 7, caractérisé en ce que les disulfures, polysulfures organiques et mercaptans ont des radicaux hydrocarbonés R choisis parmi un groupe alkyle, cycloalkyle, aryle et aralkyl.

9. Procédé suivant la revendication 8, caractérisé en ce que le radical R porte un ou plusieurs groupes fonctionnels.

10. Procédé suivant l'une des revendications 1 à 9, caractérisé en ce que la résine est présente en une quantité allant de 0,01 à 20 parties en poids pour 100 parties en poids de mélange réactionnel, résine comprise.

11. Procédé suivant l'une des revendications 1 à 10, caractérisé en ce que la réaction est effectuée à une température de - 10°C à 150°C.

12. Procédé suivant la revendication 11, caractérisé en ce que la température est de + 10°C à 120°C.

13. Procédé suivant l'une des revendications 1 à 12, caractérisé en ce que l'on ajoute du méthanol au milieu réactionnel.

## Patentansprüche

1. Verfahren zur Herstellung von organischen Disulfiden und Polysulfiden durch Einwirkung von Schwefel auf ein Thiol oder ein Polysulfid mit niedrigem Schwefelgehalt, um es in ein Polysulfid mit höherem Schwefelgehalt umzuwandeln, oder durch Einwirkung eines Mercaptans auf ein organisches Polysulfid mit hohem Schwefelgehalt, um es in ein Polysulfid mit niedrigerem Schwefelgehalt umzuwandeln, in Gegenwart eines Katalysators in Form eines basischen Harzes, dadurch gekennzeichnet, daß das Harz auf der Grundlage von Polystyrol-Divinylbenzol (PS-DVB) durch basische Gruppen der allgemeinen Formel (I) funktionell wirksam gemacht ist: 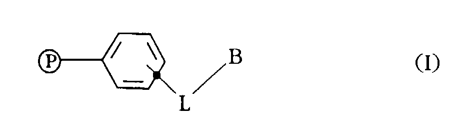 in der B eine Gruppe bedeutet, ausgewählt ist aus:
1° - einem Guanidin-Rest der allgemeinen Formel (C): 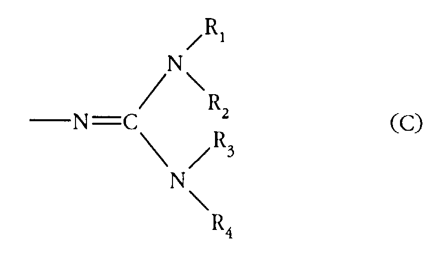 der mit L am Stickstoffimin substituiert ist, wobei R₁, R₂, R₃ und R₄ unabhängig voneinander Wasserstoff, Methyl-, Ethyl-, Propyl-, Butyl-, Cyclohexyl- und Phenylgruppen sind, mit der Bedingung, daß L dann einen organischen linearen Rest mit einer Länge gleich oder größer der Länge des Methylenrestes -CH₂- bedeutet,
2° - einem cyclischen Guanidin-Rest der Formel (D): 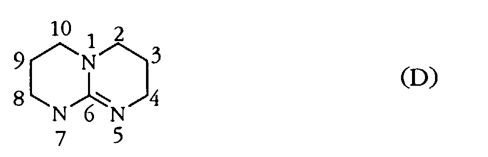 der mit L in 7-Stellung substituiert ist, mit der Bedingung, daß L dann einen -(CH₂)ₙ-Rest bedeutet, wobei n eine ganze Zahl mit dem Wert 1 oder 3 bis 9 ist,
3° - einem cyclischen Amidin der Formel (E): 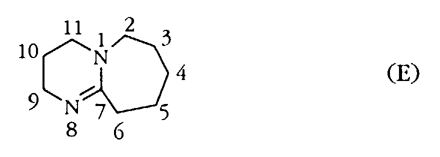 der mit L in 6-Stellung substituiert ist, mit der Bedingung, daß L dann einen -(CH₂)ₙ-Rest bedeutet, wobei n eine ganze Zahl mit dem Wert 1 oder 3 bis 9 ist,
4° - einem cyclischen Amidin der Formel (F): 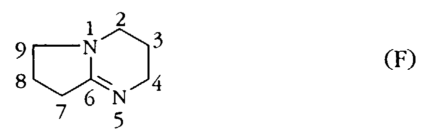 der mit L in 7-Stellung substituiert ist, mit der Bedingung, daß L dann einen -(CH₂)ₙ-Rest bedeutet, wobei n eine ganze Zahl mit dem Wert 1 oder 3 bis 9 ist,
und 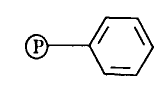 das Trägerharz PS, DVB ist.

2. Verfahren nach Anspruch 1, dadurch gekennzeichnet, daß das Harz der allgemeinen Formel (I) vom Geltyp ist.

3. Verfahren nach Anspruch 1, dadurch gekennzeichnet, daß das Harz der allgemeinen Formel (I) makrovernetzt ist und eine makroporöse Struktur hat.

4. Verfahren nach einem der Ansprüche 1 bis 3, dadurch gekennzeichnet, daß L Methylen (-CH₂-) bedeutet.

5. Verfahren nach einem der Ansprüche 1 bis 3, dadurch gekennzeichnet, daß L, wenn es an den Guanidin-Rest der Formel (C) gebunden ist, es einen Rest der Formel (II)
-CH₂-(X-CH₂-CH₂)ₘ- (II)
bedeutet, wobei X Sauerstoff (-O-) oder Schwefel (-S-) bedeutet und m 1 oder 2 ist.

6. Verfahren nach Anspruch 5, dadurch gekennzeichnet, daß X Sauerstoff bedeutet und m = 1 ist.

7. Verfahren nach Anspruch 5, dadurch gekennzeichnet, daß X Schwefel bedeutet und m = 1 ist.

8. Verfahren nach einem der Ansprüche 1 bis 7, dadurch gekennzeichnet, daß die organischen Disulfide, Polysulfide und Thiole Kohlenwasserstoffreste R haben, die aus einer Alkyl-, Cycloalkyl-, Aryl- und Aralkylgruppe gewählt sind.

9. Verfahren nach Anspruch 8, dadurch gekennzeichnet, daß der Rest R eine oder mehrere funktionelle Gruppen trägt.

10. Verfahren nach einem der Ansprüche 1 bis 9, dadurch gekennzeichnet, daß das Harz in einer Menge von 0,01 bis 20 Gewichtsteilen, bezogen auf 100 Gewichtsteile des Reaktionsgemisches einschließlich des Harzes, vorliegt.

11. Verfahren nach einem der Ansprüche 1 bis 10, dadurch gekennzeichnet, daß die Reaktion bei einer Temperatur zwischen -10 °C und 150 °C erfolgt.

12. Verfahren nach Anspruch 11, dadurch gekennzeichnet, daß die Temperatur zwischen +10 °C und 120 °C liegt.

13. Verfahren nach einem der Ansprüche 1 bis 12, dadurch gekennzeichnet, daß man dem Reaktionsgemisch Methanol zugibt.

## Claims

1. Process for the preparation of organic disulphides and polysulphides by reaction of sulphur with a mercaptan or with a polysulphide of lower sulphur order in order to convert it into polysulphide of higher order, or alternatively by reaction of a mercaptan with an organic polysulphide of high sulphur order in order to convert it into polysulphide of lower sulphur order, in the presence of a catalyst in the form of a resin with a basic function, characterized in that the resin is based on polystyrene-divinylbenzene (PS-DVB), functionalized with basic groups and having the general formula (I): 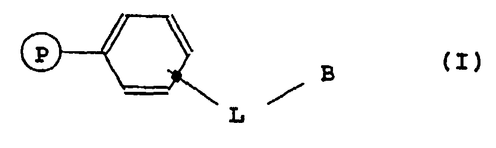 B representing a group chosen from:
1. - a guanidine radical of general formula (C): 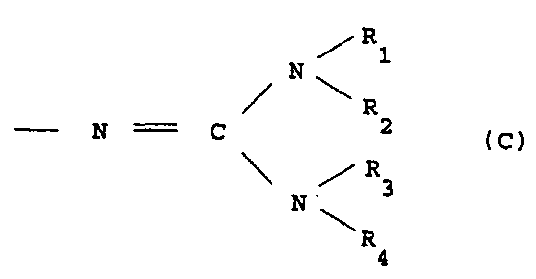 substituted with L on the imine nitrogen, in which R₁, R₂, R₃ and R₄ are, independently of each other, chosen from hydrogen and methyl, ethyl, propyl, butyl, cyclohexyl and phenyl groups, with the condition that L in this case represents a linear organic radical which is as long as or longer than the methylene radical -CH₂-,
2. - - a cyclic guanidine radical of formula (D): 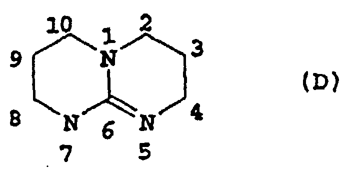 substituted with L in position 7, with the condition that L in this case represents a radical -(CH₂)ₙ-, n being an integer equal to 1 or 3 to 9,
3. - a cyclic amidine of formula (E): 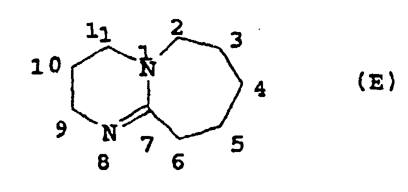 substituted with L in position 6, with the condition that L in this case represents a radical -(CH₂)ₙ-, n being an integer equal to 1 or 3 to 9,
4. - a cyclic amidine of formula (F): 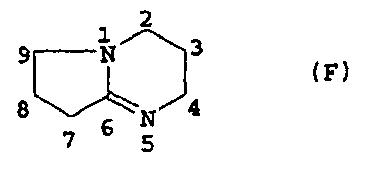 substituted with L in position 7, with the condition that L in this case represents a radical -(CH₂)ₙ-, n being an integer equal to 1 or 3 to 9 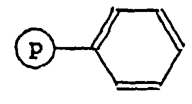 being the PS-DVB resin support.

2. Process according to Claim 1, characterized in that the resin of general formula (I) is of gel type.

3. Process according to Claim 1, characterized in that the resin of general formula (I) is macrocross-linked and of macroporous structure.

4. Process according to one of Claims 1 to 3, characterized in that L represents a methylene (-CH₂-).

5. Process according to one of Claims 1 to 3, characterized in that when L is attached to the guanidine radical of formula (C) it represents a radical of formula (II)
-CH₂-(X-CH₂-CH₂)ₘ- (II)
X representing oxygen (-O-) or sulphur (-S-) and m is equal to 1 or 2.

6. Process according to Claim 5, characterized in that X represents oxygen and that m is equal to 1.

7. Process according to Claim 5, characterized in that X represents sulphur and m is equal to 1.

8. Process according to one of Claims 1 to 7, characterized in that the mercaptans and organic disulphides and polysulphides have hydrocarbon radicals R chosen from an alkyl, cycloalkyl, aryl and aralkyl group.

9. Process according to Claim 8, characterized in that the radical R bears one or more functional groups.

10. Process according to one of Claims 1 to 9, characterized in that the resin is present in an amount ranging from 0.01 to 20 parts by weight per 100 parts by weight of reaction mixture, including resin.

11. Process according to one of Claims 1 to 10, characterized in that the reaction is carried out at a temperature of from -10°C to 150°C.

12. Process according to Claim 11, characterized in that the temperature is from +10°C to 120°C.

13. Process according to one of Claims 1 to 12, characterized in that methanol is added to the reaction medium.
